# EUROPÄISCHE PATENTANMELDUNG

(11) **EP 2 311 416 A1**
(43) Veröffentlichungstag der Anmeldung: **20.04.2011**
(21) Anmeldenummer: 10013577.1
(22) Anmeldetag: 11.10.2010
(51) Int. Cl.: A61F 11/08, H04R 25/00

(54) **Dämmkommunikationsplastik mit Elektronik für Personen mit Hörminderung am Lärmarbeitsplatz**

(30) Priorität: 16.10.2009 DE 202009014016 U
(71) Anmelder: Meyer, Thomas, 91217 Hersbruck (DE)
(72) Erfinder: Meyer, Thomas, 91217 Hersbruck (DE)

(57) **Zusammenfassung**

Es wird ein spezieller Gehörschutz (1) in Verbindung mit einem Hörsystem (6) vorgeschlagen. Der Gehörschutz zeichnet sich dadurch aus, dass er eine integrierte Schalleitung (2) zur Anbindung eines Hörsystems aufweist.

## Beschreibung

### Einleitung

Menschen, die einen Arbeitsplatz in lauter Umgebung haben, müssen ab einem Lärmpegel von 80dB einen Gehörschutz tragen.

Hat aber diese Person bereits einen Hörverlust, wird beim Tragen des Gehörschutzes das Gehör so beeinflusst, dass Signaltöne oder eine Grundkommunikation nicht mehr wahrnehmbar sind.

Trägt der Betroffene sein Hörgerät am Arbeitsplatz wird alles noch viel lauter und sein Gehör wird noch mehr geschädigt.

Dazu kommt das die dazugehörige Otoplastik keine Dämmwirkung aufweist, unter Umständen sogar noch eine Verstärkende Wirkung der Schallweiterleitung hat.

An einem Lärmarbeitsplatz kann und darf kein Standarthörsystem- Gerät eingesetzt werden. Hierfur gibt es bislang keine Lösung, der Arbeitnehmer kann in der Regel diesen Arbeitsplatz nicht mehr ausüben.

### Beschreibung

### Gebiet der Erfindung

Die vorliegende Erfindung bezieht sich auf ein Dämmkommunikationsplastik und insbesondere auf ein Ohrteil welches den Ohrkanal Dämmt und vor Lärm schützt. Welches aber in Verbindung mit einer entsprechenden Elektronik in der Lage ist Geräusche zu kompensieren die noch im Hörkanal ankommen müssen.

### Stand der Technik

Der bisher am Markt bestehende Gehörschutz kann nur eine Bestimmte Dämmwirkung erzielen, je nach Bauart, Material und Filter.

Der Schall wird Gedämmt und soll den Ohrkanal nicht erreichen.

Es ist nicht möglich, am Lärmarbeitsplatz, dem Ohrkanal, Schall über eine Schalleitung zuzuführen und dabei aber das Ohr, vor Lärm, gleichzeitig zu schützen. Diese Art von Gehörschutzdämmplastik kann nur für den normal hörenden eingesetzt werden.

### Die Erfindung

Der im Schutzanspruch 1 angegebenen Erfindung liegt das Problem zugrunde, eine speziell gefertigte Dämmplastik in Verbindung mit einem Hörsystem, welches technisch den Anforderungen gerecht wird zu entwickeln.

Die Dämmplastik erfüllt ihre Aufgabe, um den Schall zu vermindern. Das Hörsystem verstärkt die lauten Pegel gar nicht, es werden nur die leisen Pegel sowie Anteile der Sprachlautstärke über eine Schalleitung direkt an das Gehör weiter geleitet.

Das Hauptproblem in der Vergangenheit war die Hörgerätetechnik, der Fortschritt dieser Technik ermöglicht es eine Kombination herzustellen, das Besondere ist es das System so aufeinander abgestimmt ist, dass der nur der Nutzschal im Ohrkanal ankommt und das Ohr nicht geschädigt wird.

Das System wird hierbei speziell aufeinander abgestimmt und erfüllt daher die erforderlichen Eigenschaften, der Ausgansschallpegel wird eine Höhe die für das Ohr schädigend sein kann nicht überschreiten.

Die Überprütbarkeit des Schallpegels am Ohr wird durch einen Anschluss für ein Prüfmikrofon oder einen Insitumessschlauch gewährleistet.

Das System wird mehrer Programm die mittels Taster oder einer Fernbedienung einstellbar sind haben, hiermit können verschiedene Arbeitsbereicht bewältigt werden.

### Beispiel:

Besprechung im Büro Programm 1 , der Betroffene kann der Besprechung Folgen und die Sprache sogar mit der FB etwas lauter stellen.

Werkshalle mit durchschnittlich 87 dB Lautstärke, Programm 2, der Betroffene wird vom Lärm geschützt, ist aber für Kollegen ansprechbar, da leise wie mittlere Pegel entsprechend über das Dämmkommunikationssystem ans Ohr gegeben werden, dies gilt auch für entsprechende Signaltöne.

Telefoneingangin der Werkshalle, das System schaltet automatisch um und der Betroffene kann den Anrufer direkt im Dämmkommunikationssystem hören.

Für beides in Kombination gibt es bei zuständigen Stelle das BGIA, das Institut für Arbeitsschutz in Sankt Augustin noch keine Zulassung.

Wir sind seit April 2010 der erste Hersteller im entstehenden Zulassungsverfahren für diese Art von Dämmkommunikationsplastik für Hörgeschädigte am Lärmarbeitsplatz.

Das Zulassungsverfahren wurde in Zusammenarbeit der Berufsgenossenschaft sowie der Prüfstelle und meiner Person Thomas Meyer entwickelt.

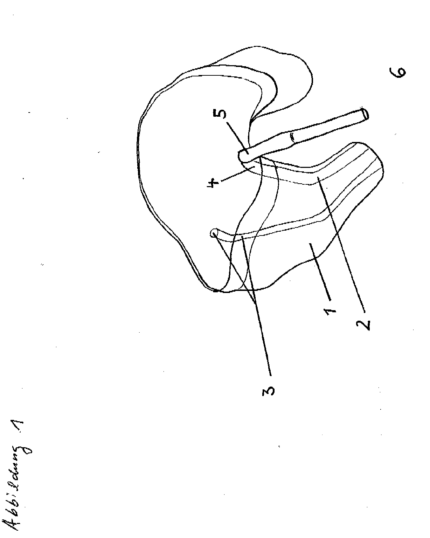

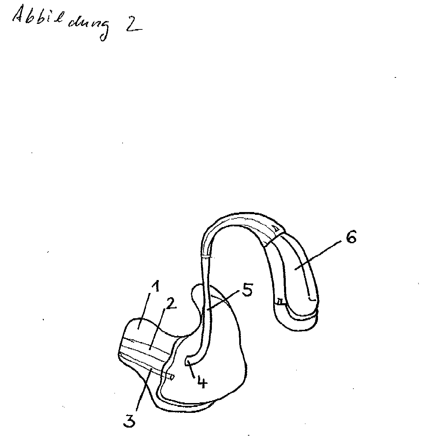

## Patentansprüche

1. Dämmkommunikationsplastik mit Unterdrückung von Lärm im Ohrkanl, welches umfasst: eine Dämmplastik mit kunststoffähnlichen Otoplastik/ Dämmplastik (1),welche auch als Hohlplastik oder Vollplastik sowie in Gehörgangs oder Concha Form gefertigt werden kann. Sie wird im Ohrkanal untergebracht um laute Pegel nicht in den Ohrkanal eindringen zu lassen; eine integrierte Schallleitung (2)oder Anbindung eines externen Hörers ( Lautsprechers ) in der Dämmplastik; eine Anbindung für ein Prüfmikrofon oder Insituprüfschlauch (3), welche das System der Möglichkeit zur Überprüfung des Schallpegels im Gehörgang und somit die Funktion gewährleistet werden kann.

2. Dämmkommunikationsplastik **dadurch gekennzeichnet, dass** ein intekrieter Schallschlauchzugang oder in Form eines elektronischen Zugang (4) gegeben ist und das System in sich geschlossen bleibt und die Dämmwirkung nicht verändert werden kann.

3. Dämmkommunikationsplastik **dadurch gekennzeichnet**, wobei die Filtermittel von 63 Hz bis 8000 Hz eine Dämmwirkung nach europäischen Norm EN352-2 erfüllen und eine **Baumusterprüfbescheinigung besitzt**.

4. Dämmkommunikationsplastik **dadurch gekennzeichnet**, Steckverbindungsmittel (5) zum Verbinden der Dämmplastik mit der elektronischen- technischen Einheit

5. Dämmkommunikationsplastik **dadurch gekennzeichnet**, das die elektronische- technische Einheit in der Dämmkommunikationsplastik sowie ausgelagert (6) bereit gestellt ist. Dies kann in Form eines Gehäuses hinter dem Ohr sowie zum umhängen oder einstecken erfolgen.

6. Dämmkommunikationsplastik **dadurch gekennzeichnet, dass** bei der elektronisch- technischen Einheit der Ausgangsschalldruckpegel begrenzt werden kann. Dazu kommt die Möglichkeit die leisen, mittleren und lauten Pegel frequenzspezifisch einstellen zu können, auch sind mindestens 2-6 programmierbare Hörprogramme einzusetzen.
